(19) Europäisches Patentamt

European Patent Office

Office européen des brevets

(11) Veröffentlichungsnummer: **0 238 064 B1**

# (12) EUROPÄISCHE PATENTSCHRIFT

(45) Veröffentlichungstag der Patentschrift: **29.07.92**

(51) Int. Cl.⁵: **A61B 5/02**

(21) Anmeldenummer: **87103980.6**

(22) Anmeldetag: **18.03.87**

(54) **Einrichtung zur nichtinvasiven Feststellung und akustischen Darstellung des dynamischen Verhaltens der peripheren venösen Hämodynamik.**

(30) Priorität: **18.03.86 DE 3609073**

(43) Veröffentlichungstag der Anmeldung:
**23.09.87 Patentblatt 87/39**

(45) Bekanntmachung des Hinweises auf die
Patenterteilung:
**29.07.92 Patentblatt 92/31**

(84) Benannte Vertragsstaaten:
**AT BE CH DE ES FR GB GR IT LI LU NL SE**

(56) Entgegenhaltungen:
**EP-A- 0 063 649**
**DE-A- 3 409 792**
**US-A- 4 325 383**
**US-A- 4 591 729**

(73) Patentinhaber: **A. Nattermann & Cie. GmbH**
**Nattermannallee 1**
**W-5000 Köln 30(DE)**

(72) Erfinder: **Schmitt, Hans J., Prof. Dr. rer. nat.**
**Hangstr.34**
**W-5100 Aachen(DE)**
Erfinder: **Blazek, Vladimir, Dr.-Ing.**
**Koenigshuegel 31**
**W-5100 Aachen(DE)**

(74) Vertreter: **Schiller, Walter, Dr. et al**
**Willibaldstrasse 36**
**W-8000 München 21(DE)**

## Beschreibung

Die Erfindung bezieht sich auf eine Meßeinrichtung zur nichtinvasiven Feststellung peripherer Abfluß- und Durch-flußstörungen in menschlichen Extremitäten gemäß dem Ober-begriff des Patentanspruchs 1.

Meßeinrichtungen dieser Art sind beispielsweise aus dem deutschen Patent 31 00 610.8 oder dem deutschen Patent 33 18 746.0 bekannt.

Bei der aus der DE-PS 31 00 610.8 bekannten Meßeinrichtung wird der zeitliche Verlauf des reflektierten bzw. zurück-gestreuten Strahlungsanteils analog ausgewertet und mit-tels eines Schreibers aufgezeichnet.

Bei der aus der DE-PS 33 18 746.0 bekannten Meßeinrichtung wird das analoge Signal mittels einer Schnittstellenschaltung in ein digitales Signal umgesetzt und an einen Rechner angelegt. Der Rechner berechnet physikalische Bewertungsparameter für die analogen Lichtreflexions-Kurven. Diese Meßeinrichtung ist damit insbesondere zur Durchführung und Auswertung von Reihenuntersuchungen geeignet.

Beiden bekannten Meßeinrichtungen ist der Nachteil gemein-sam, daß sie kaum als handliches, tragbares Gerät ausführ-bar sind: weder der gemäß der DE-PS 31 00 610.8 verwendete Schreiber noch der gemäß der DE-PS 33 18 746.0 verwendete handelsübliche Mikrocomputer mit Floppy-Disc-Laufwerken usw. sind soweit miniaturisierbar, daß sie in ein tragbares Gerät problemlos einbaubar sind. Darüberhinaus ist auch der Stromverbrauch der bekannten Meßeinrichtungen für tragbare Geräte zu groß.

Besonders nachteilig ist es jedoch, daß der Beginn und das Ende einer jeden Messung von der Bedienungsperson festzulegen sind, so daß subjektive Meßfehler möglich sind.

Der Erfindung liegt die Aufgabe zugrunde, eine Meßeinrich-tung zur nichtinvasiven Feststellung peripherer Abfluß-und Durchflußstörungen in menschlichen Extremitäten anzu-geben, die auch als tragbares Handgerät ausführbar ist.

Eine erfindungsgemäße Lösung dieser Aufgabe ist mit ihren Weiterbildungen in den Patentansprüchen angegeben.

Überraschenderweise gelingt eine Lösung dieser Aufgabe da-durch, daß weiterhin von einer Meßeinrichtung gemäß Ober-begriff des Patentanspruchs 1 ausgegangen und diese Meß-einrichtung durch die im kennzeichnenden Teil des Patent-anspruchs 1 angegebenen Merkmale weitergebildet wird. Der Erfindung liegt dabei der Grundgedanke zugrunde, daß die Auswerteschaltung einen digital gesteuerten Tongenerator aufweist, und die Ausgabe der Meßergebnisse mittels eines elektroakustischen Wandlers erfolgt. Hierdurch ist es möglich, die Meßeinrichtung als kompaktes und leichtes Ge-rät mit geringem Stromverbrauch auszuführen. Der Arzt kann damit die erfindungsgemäße Meßeinrich-tung beispielsweise bei einem Bewegungspro-gramm, bei dem sportliche Übungen etc. durchgeführt werden, ohne weiteres mit sich führen und sich unmittelbar nach Abschluß der Übung ohne größeren Aufwand von dem Auffüll- bzw. Entleerverhalten der Venen überzeugen. Hierzu gibt die Auswerte- und Ausgabeschaltung ein erstes Signal, das beispielsweise ein optisches Aufmerksamkeitssignal, bevorzugt jedoch ein Ton ist (Anspruch 2), ab, der der Untersuchungsperson anzeigt, daß die Einrichtung zur Messung bereit und eine konstante Ruhedurchblutung der Haut gegeben ist. Das eigentliche Meßergebnis, d.h. die gemessene Lichtreflexion wird durch eine zweite Tonfolge dargestellt, deren Frequenz der Änderung der Intensität der Lichtreflexion - z.B. als Folge von Beinbewegungen - bis zum Ende der Blutentleerung bzw. zur Auffüllung folgt. Das Ende der Messung, d.h. das Wiedererreichen einer konstanten Hautdurchblutung nach erfolgter Bewegung, wird dann durch ein drittes Signal, wiederum bevorzugt einen dritten Ton bzw. eine dritte Tonfolge -gegebenenfalls auch durch das Ende des Tons - angezeigt.

Diese akustische Ausgabe des Meßergebnisses ermöglicht es der Untersuchungsperson - wie sich überraschenderweise herausgestellt hat - sehr gut, die Entleerung bzw. Auffül-lung der Venen oder Arterien zu analysieren und krankhafte Veränderungen etc. zu erkennen.

Gemäß Anspruch 3 ist es ferner möglich, den Tongenerator und den erfindungsgemäß vorgesehenen elektroakustischen Wandler - beispielsweise einen Lautsprecher - dazu zu ver-wenden, das Bewegungsprogramm der zu untersuchenden Person beispielsweise durch eine rhythmische Tonfolge zu unter-stützen. Selbstverständlich ist es möglich, zwischen ver-schiedenen Tonfolgen zu wählen, die unterschiedliche Bewe-gungsprogramme unterstützen.

Die erfindungsgemäße Einrichtung kann beispielsweise eine Selbsteichung durchführen, bei der die dem Lichtsender bzw. den Lichtsendern zugeführte Energie kontinuierlich solange erhöht wird, bis das vom Lichtempfänger bzw. von den Lichtempfängern gemessene Signal einen bestimmten Signal/Rausch-Abstand hat. Der erste Ton, der den Beginn der Meßbereitschaft angibt, kann dann nach Beendigung der Selbsteichung abgegeben werden.

Die erfindungsgemäße Einrichtung kann nach Anspruch 6 na-türlich auch einen Speicher sowie einen Datenausgang auf-weisen. In dem Speicher können die Meßergebnisse gespei-chert und dann an ein anderes Auswertegerät übergeben wer-den. Selbstverständlich ist es aber auch möglich, über

eine Datenleitung während der Messung die Meßergebnisse und/oder die Auswerergebnisse an ein weiteres Auswertegerät, beispielsweise einen Mikrocomputer oder einen Drukker zu übertragen.

Die Erfindung wird nachstehend anhand von Ausführungsbeispielen unter Bezugnahme auf die Zeichnung näher beschrieben, in der zeigen:

Fig. 1 schematisch eine erfindungsgemäße Meßeinrichtung,

Fig. 2 ein Blockschaltbild einer erfindungsgemäß verwendeten Auswerte- und Ausgabeschaltung,

Fig. 3 eine Erläuterung der erfindungsgemäß gewählten Darstellung der Meßergebnisse,

Fig. 4 die Anzeigen bei der in Fig. 3 dargestellten akustischen Darstellung, und

Fig. 5 eine weitere Möglichkeit der akustischen Darstellung.

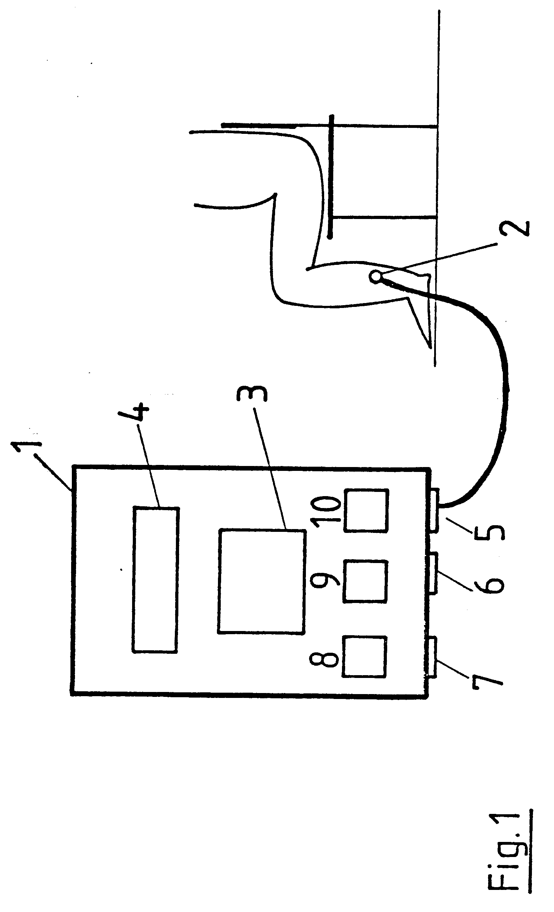

Fig. 1 zeigt schematisch eine erfindungsgemäße Meßeinrichtung 1, die als kleines, tragbares Gerät ausgeführt ist. Die Meßeinrichtung 1 weist einen elektroakustischen Wandler 3, beispielsweise einen Lautsprecher, eine LCD-Anzeigeeinheit 4, einen Stecker 5 für einen optischen Meßkopf 2 mit einem oder mehreren Lichtsendern und -empfängern, der an einer menschlichen Extremität anbringbar ist, einen Stecker 6 für einen Kopfhörer sowie einen Stecker 7 für die Datenübertragung zu einem stationären Auswertegerät auf. Mit 8 ist ein Ein/Aus-Schalter, mit 9 eine Funktionstaste zum Wählen verschiedener Betriebsarten und mit 10 eine Anzeigetaste (z.B. für die Menüführung) bezeichnet.

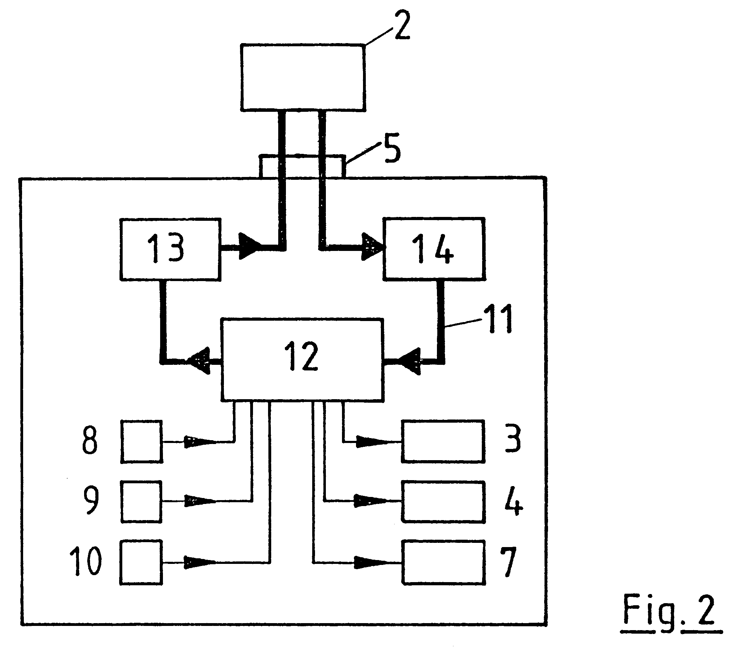

Fig. 2 zeigt ein Blockschaltbild der erfindungsgemäßen Auswerte- und Ausgabeschaltung. Dabei sind gleiche Elemente wie in Fig. 1 mit denselben Bezugszeichen bezeichnet. Ein Mikroprozessor 12, ein Treiber 13 für den Lichtsender und ein Empfänger 14 für den Lichtempfänger bilden eine aktive Regelschleife 11. Der Treiber 13 und der Empfänger 14 können dabei in ähnlicher Weise wie in den deutschen Patentschriften 31 00 610 und 33 18 746 beschrieben aufgebaut sein. Auf diese Patentschriften wird im übrigen zur Erläuterung aller hier nicht im einzelnen erläuterten Begriffe ausdrücklich Bezug genommen.

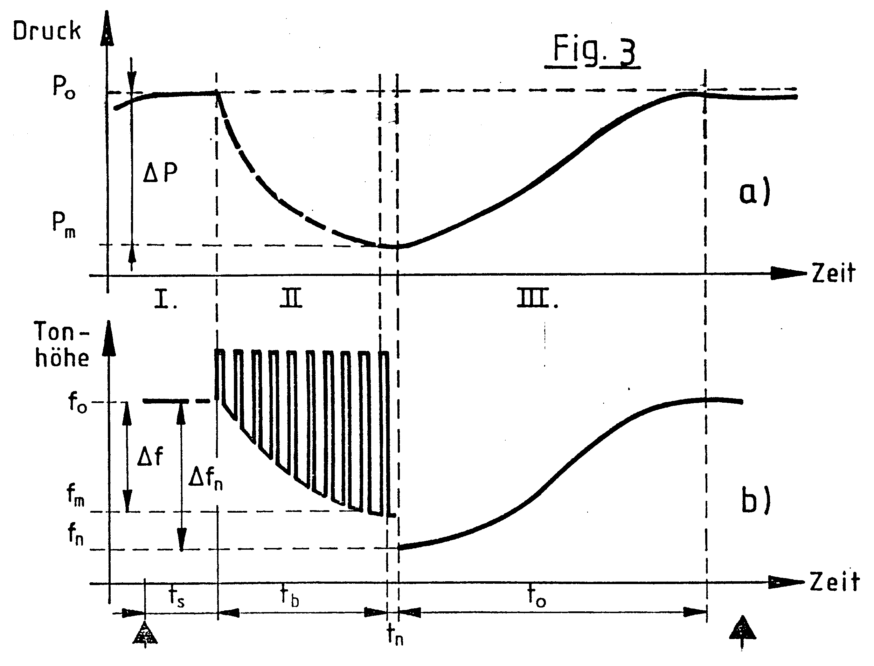

Die Funktionsweise der in den Fig. 1 und 2 dargestellten Meßeinrichtung wird im folgenden unter Bezugnahme auf die Fig. 3 bis 5 näher erläutert.

Fig. 3a zeigt ein Beispiel für eine Venendruckkurve, wie sie invasiv mittels der sogenannten Phlebo-Dynamometrie erhalten wird. In der Phase I wird bei sitzendem Patienten eine konstante Hautdurchblutung und damit ein nahezu konstanter Druck $P_o$ erreicht. In der Phase II führt der Patient Bewegungen aus. Demgemäß fällt der Druck vom

Wert $P_o$ auf den für den jeweiligen Zustand der Gefäße charakteristischen Druck $P_m$ ab. Nach Beendigung der Bewegungsphase steigt der Druck in der Phase III wieder in etwa auf den ursprünglichen Wert $P_o$ an. Die Zeitdauer, in der dies geschieht, ist ebenfalls ein Hinweis auf den jeweiligen Gefäßzustand.

Wie ausführlich beispielsweise in der DE-PS 31 00 610 dargestellt, folgt die Lichtreflexion der in Fig. 3a dargestellten Druckkurve.

Fig. 3b zeigt die akustische Ausgabe, die man bei der in Fig. 3a dargestellten Druckkurve mit der erfindungsgemäßen Meßeinrichtung erhält.

Nach Erreichen der konstanten Hautdurchblutung bei sitzendem Patienten und durchgeführter Selbsteichung wird ein Ton mit der Frequenz $f_o$ für eine Dauer von beispielsweise 10 Sekunden abgegeben. Vor Beginn der ersten Bewegungsphase wird dieser Ton kurz unterbrochen. Diese Unterbrechungen können beispielsweise 2 Sekunden und 1 Sekunde vor Beginn der ersten Bewegung der Bewegungsphase liegen, und sind ein Hinweis für die untersuchte Person, daß das Bewegungsprogramm beginnt. In der Bewegungsphase II wird während der Zeitdauer $t_b$ eine Tonfolge abgegeben, deren Frequenz entsprechend dem Druckabfall von $P_o$ auf $P_m$ von $f_o$ auf $f_m$ sinkt. Nach Beendigung der Bewegungsphase II wird die Frequenz des abgegebenen Tones von der für jede untersuchte Person individuellen Frequenz $f_m$ auf eine Standardfrequenz $f_n$ normiert, deren Wert beispielsweise 250 Hz ($f_o/4$) ist. Die Startfrequenz $f_o$ ist 1000 Hz. Diese Frequenz ist bevorzugt, da das menschliche Ohr Frequenzänderungen in diesem Bereich am besten unterscheiden kann.

Während der Auffüllphase III steigt die Frequenz von dem Wert $f_n$ auf den Ausgangswert $f_o$. Am Ende der Auffüllphase wird ein Ton konstanter Frequenz während einer Zeit von beispielsweise 10 Sekunden abgegeben. Anschließend wird die Messung beendet. Das Ende der Messung wird durch die Beendigung des Tons der Untersuchungsperson und vorzugsweise auch dem Untersuchten angezeigt.

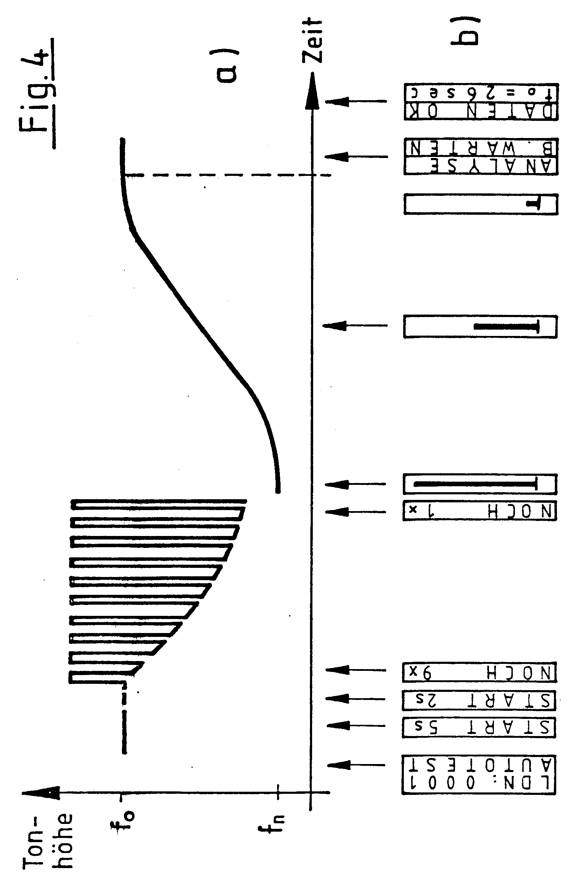

In Fig. 4b ist die Anzeige auf der Anzeigeeinheit 4 während der Messung dargestellt. Fig. 4a entspricht dabei Fig. 3b, so daß auf eine nähere Erläuterung von Fig. 4a verzichtet werden kann.

Die Anzeigeeinheit 4 enthält zu Beginn der Messung eine Information darüber, daß zur Zeit ein Selbsttest durchgeführt wird, bzw. wieviel Zeit bis zum Beginn der Messung noch vergeht. Anschließend zeigt sie die Zahl der noch während des Bewegungsprogramms zu hörenden Töne und damit die Restdauer des Bewegungsprogramms an. Während der Auffüllphase kann sie beispielsweise durch Darstellung eines Balkens die momentane Hautreflexion quasi analog anzeigen. Nach Beendigung der Auffüllphase zeigt sie nach einer kurzen Analysezeit z.B. die Zeitdauer $t_o$ an, die zur Auffüllung benötigt worden ist. Die Umschaltung auf verschiedene andere Anzeigewerte erfolgt mittels der Anzeigetaste 10. Damit unterstützt die Anzeigeeinheit einerseits die akustische Ergebnisausgabe und kann andererseits zu einem interaktiven Dialog über Menüführung verwendet werden.

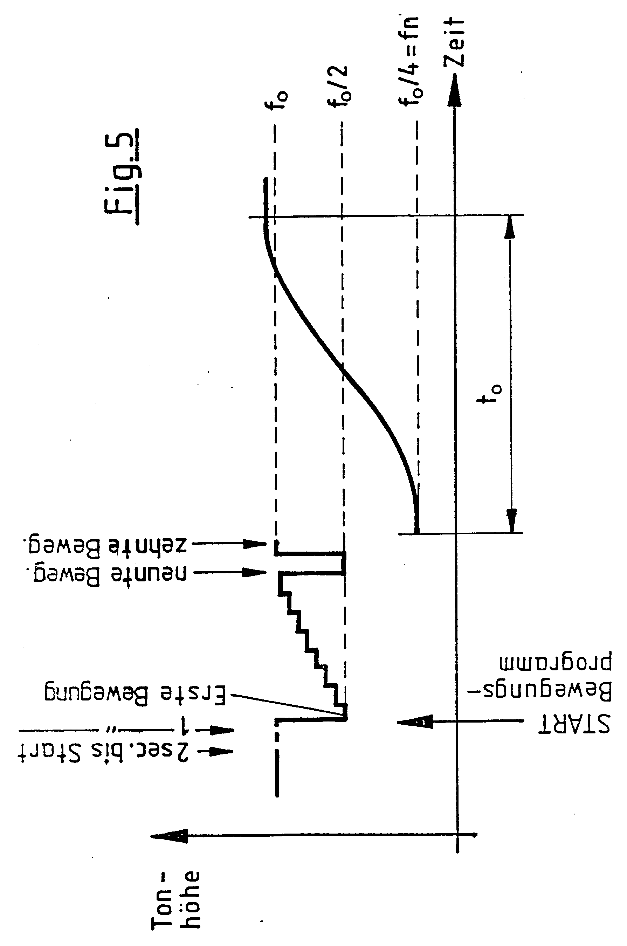

Fig. 5 zeigt ein weiteres Beispiel für die erfindungsgemä-ße akustische Ausgabe der Meßergebnisse. Bei diesem Bei-spiel wird während der Bewegungsphase die untersuchte Per-son durch die über den Lautsprecher 3 abgegebene Tonfolge geführt. Dabei können die Töne beispielsweise über eine Oktave variieren. Der Beginn der Auffüllphase wird durch eine Veränderung des Tons um zwei Oktaven zu tieferen Fre-quenzen hin angezeigt, wobei der Startton $f_o$ wiederum vor-zugsweise bei 1 kHz liegt. Die Auffüllphase wird wiederum durch eine Änderung der Tonhöhe dargestellt.

Vorstehend ist die Erfindung anhand von Ausführungsbeispielen beschrieben worden. Innerhalb des allgemeinen Er-findungsgedankens sind natürlich die verschiedensten Modifikationen möglich.

Beispielsweise ist es möglich, den Patienten über die Lautsprechereinheit 3 akustisch zu führen, während für die Untersuchungsperson die Blutauf-füllung bzw. -entleerung mittels Kopfhörer darge-stellt wird.

Die Frequenz f der erzeugten Töne kann sich natürlich auch gegenläufig zur Druckänderung verschieben.

Ferner ist es mit Hilfe der Anzeigetaste 10 möglich, nach dem Ende einer jeden Messung weitere aus den gemessenen und gespeicherten Meßdaten berechnete Parameter zur Anzeige zu bringen. Mit Hilfe der Funktionstaste 9 können beispielsweise folgende im Programmspeicher des Mikrocomputers 12 gspeicherte Programmpakete angewählt werden:
- Standard-Meßprogramm - Daten-Ausgabeprogramm - Lern- und Schulprogramm für Bedienungspersonen.

**Patentansprüche**

1. Meßeinrichtung (1) zur nichtinvasiven Feststel-lung peripherer Abfluß- und Durchflußstörun-gen in menschlichen Extremitäten, die zur Erfassung des zeitlichen Verlaufs der Blutentleerung bzw. -auffüllung der Venen durch Messung der Änderung der Lichtreflexion mindestens einen Lichtsender und einen -empfänger sowie eine Auswerte- und Ausgabeschaltung aufweist,
dadurch **gekennzeichnet**, daß die Auswerte- und Ausgabeschaltung einen digital gesteuerten Tongenerator und einen elektroakustische Wandler (3) aufweist und
- ein erstes Signal zur Anzeige der Meßbereitschaft der Einrichtung,
- ein zweites Signal bestehend aus einer Tonfolge, deren Frequenz der Intensitätsänderung der Lichtreflexion bis zum Ende der Blutentleerung bzw. Auffüllung folgt, und
- ein drittes Signal abgibt, das das Ende der Messung anzeigt.

2. Einrichtung nach Anspruch 1,
dadurch **gekennzeichnet**, daß das erste und das dritte Signal akustische Signale sind.

3. Einrichtung nach Anspruch 1 oder 2,
dadurch **gekennzeichnet**, daß zur Unterstüt-zung des Bewegungsprogramms der Tongene-rator zwischen dem ersten Ton und der zweiten Tonfolge eine weitere Tonfolge abgibt.

4. Einrichtung nach einem der Ansprüche 1 bis 3,
dadurch **gekennzeichnet**, daß in die zweite Tonfolge zusätzlich eine Folge kurzer Impulse zur Verbesserung der zeitlichen Information integriert ist.

5. Einrichtung nach einem der Ansprüche 1 bis 4,
dadurch **gekennzeichnet**, daß die Einrichtung vor Beginn der Messung eine Selbsteichung durchführt, und der Tongenerator den ersten Ton nach Beendigung der Selbsteichung abgibt.

6. Einrichtung nach einem der Ansprüche 1 bis 5,
dadurch **gekennzeichnet**, daß die Einrichtung einen Speicher zur digitalen Speicherung der Meßergebnisse aufweist.

**Claims**

1. Non-invasive device (1) for determining malfunction in the peripheral discharge and passage in human extremities, which comprises at least one light emitter and one light receiver for the detection of the course of blood discharge from or blood supply to the venous vessels by means of measuring the change of light reflexion, as well as one evaluation and output circuit,
**characterized** in that said evaluation and output circuit comprises a digitally controlled sound generator and an electroacoustic converter (3), and emits
- a first signal for indicating readiness of the device for measuring operation,
- a second signal consisting of a succession of sounds whose frequency follows the variation of the intensity of the light reflexion up to the end of blood discharge or supply, and

- a third signal to indicate the end of measurement.

2. Device according to Claim 1, **characterized** in that said first and third signals are acoustic signals,

3. Device according to Claim 1 or 2, **characterized** in that said sound generator outputs a further succession of sounds between said first sound and said second succession of sounds so as to support the program of movement.

4. Device according to any of Claims 1 to 3, **characterized** in that an additional succession of short pulses is integrated into said second succession of sounds so as to improve the time-related information.

5. Device according to any of Claims 1 to 4, **characterized** in that the device performs an automatic self-calibration prior to the commencement of the measuring operation, and that said sound generator issues the first sound upon termination of self-calibration.

6. Device according to any of Claims 1 to 4, **characterized** in that the device comprises a memory for digital storing of the results of measurement.

**Revendications**

1. Dispositif de mesure pour l'étude non envahissante de troubles d'écoulement et de passage du sang dans la périphérie des extrémités humaines, comprenant au moins un émetteur et un récepteur de lumière pour saisir le déroulement temporel de l'évacuation ou du remplissage de sang des veines en mesurant des variations de réflexion de lumière, ainsi qu'un circuit d'évaluation et de sortie, **caractérisé** en ce que ledit circuit d'évaluation et de sortie comprend un générateur de son à commande digitale, et un convertisseur électroacoustique, et émet

- un premier signal indicant que le dispositif est prêt à mesurer,
- un deuxième signal composé d'une suite de sons dont la fréquence suit les variations d'intensité de la réflexion de lumière jusqu'à la fin de l'évacuation ou de remplissage de sang, et
- un troisième signal indicant la fin du mesurage.

2. Dispositif selon la Revendication 1, **caractérisé** en ce que les premier et troisième signaux sont des signaux acoustiques.

3. Dispositif selon la Revendication 1 ou 2, **caractérisé** en ce que ledit générateur de son émet, entre le premier son et la deuxième suite de sons, une autre suite de sons de manière à soutenir le programme de mouvement.

4. Dispositif selon une quelconque des Revendications 1 à 3, **caractérisé** en ce qu'une suite supplémentaire de courtes impulsions est intégrée à la deuxième suite de sons de manière à améliorer l'information temporel.

5. Dispositif selon une quelconque des Revendications 1 à 4, **caractérisé** en ce que le dispositif accomplit un auto-étalonnage avant le commencement du mesurage, et en ce que ledit générateur de son émet le premier son dès que l'auto-étalonnage est terminé.

6. Dispositif selon une quelconque des Revendications 1 à 5, **caractérisé** en ce que le dispositif comprend une mémoire destinée à la mémorisation digitale des résultats de mesure.

Fig.1

Fig. 2

Fig. 3

# Fig.4

a)

Zeit

b)

Ton-höhe

$f_o$

$f_n$

DATEN OK
$t_o = 26\,sec$

ANALYSE
B. WARTEN

NOCH 1x

NOCH 9x

START 2s

START 5s

AUTOTEST
LDN: 0001

Ton-höhe

2 sec. bis Start

Erste Bewegung

neunte Beweg.

zehnte Beweg.

$f_o$

$f_o/2$

$f_o/4 = f_n$

Zeit

$t_o$

START Bewegungs-programm

Fig. 5

EP 0 238 064 B1